# EUROPEAN PATENT APPLICATION

(11) **EP 1 589 021 A1**
(43) Date of publication of application: **26.10.2005**
(21) Application number: 03768157.4
(22) Date of filing: 24.12.2003
(51) Int. Cl.: C07H 1/00, C07H 3/02, C07D 307/33

(54) **PROCESS FOR PRODUCING 2-DEOXYALDOSE COMPOUND**

(30) Priority: 26.12.2002 JP 2002377740
(71) Applicant: Mitsui Chemicals, Inc., Tokyo 105-7117 (JP)
(72) Inventor: UMETANI, Hideki Mitsui Chemicals, Inc., Sodegaura-shi Chiba 299-0265 (JP); KOMATSU, Hironori Mitsui Chemicals, Inc., Sodegaura-shi2 Chiba 299-0265 (JP); ANDO, Tomoyuki Mitsui Chemicals, Inc., Sodegaura-shi Chiba 299-0265 (JP); TOGASHI, Kazuhiko Mitsui Chemicals, Inc., Sodegaura-shi Chiba 299-0265 (JP)
(74) Representative: Paget, Hugh Charles Edward
(86) International application number: PCT/JP2003/016567
(87) International publication number: WO 2004/058785

(57) **Abstract**

An object of the present invention is to provide a method for preparing 2-deoxyaldoses on industrial scale in which the yield or the volumetric efficiency is excellent and the operation is simple, as compared to the conventionally known preparation method.

A compound represented by the general formula (1) such as 2-keto-3-deoxygluconic acid or the like is reduced by the catalytic hydrogenation method using a metal such as palladium or the like, or a compound represented by the general formula (1) such as 2-keto-3-deoxygluconic acid or the like is reduced by using a hydride reducing agent in a solvent of not more than 30 weight times the amount of the above compound, for synthesizing 2-keto-3-deoxyaldonic acid. The 2-keto-deoxyaldonic acid was decarboxylated to obtain 2-deoxyaldoses.

The method of the present invention is economical and efficiently excellent.

## Description

### TECHNICAL FIELD

The present invention relates to a method for preparing 2-deoxyaldoses.

### BACKGROUND ART

In order to proceed with a reaction to be targeted in synthesizing saccharide, there have been shown many preparation methods in which a protecting group was attached to a hydroxyl group contained in a compound. In the synthesis of 2-deoxyaldoses, there has been reported a method of synthesis in which a hydroxyl group was protected with an acyl group or acetals. However, such a method requires deprotection or purification so that the number of steps increases; therefore, it is not desirable on industrial scale. Accordingly, a preparation route which does not employ a protecting group is considered favorable as a preparation method on industrial scale. From this point of view, as a representative example of a conventional technique, there can be exemplified a method comprising alkali decomposition of glucose, followed by the acidification of the solution to obtain metasaccharic acid lactone, then hydrolysis of the obtained metasaccharic acid lactone, and further decomposition reaction of the hydrolyzed product by iron to finally obtain 2-deoxyriboses (J. Am. Chem. Soc., vol. 76, p. 3541 (1954). However, alkali decomposition of glucose involves a complicated reactive system so that the yield up to metasaccharic acid is low. For this reason, the total yield up to 2-deoxyribose is only about 5%, which is not quite satisfactory as a preparation method on industrial scale.

On the other hand, as a known technique of 3-deoxyaldonic acids such as metasaccharic acid or the like obtained in a reduction step of the present invention, there has been known a method of synthesizing from D-glucono-1,5-lactone or the like which was exemplified in Acta Chim. Scand., vol. B35, p. 155 (1981) or the like. However, this method requires the protection of a hydroxyl group. Thus, this method is not desirable on industrial scale, either. Furthermore, in Carbohydr. Res., vol. 115, p. 288 (1983) was also exemplified a method of reducing potassium 2-keto-3-deoxy-D-gluconate using sodium borohydride. This method had problems such that not only the cost was high but also sodium borohydride for generating hydrogen by decomposition was used in a highly excess amount (11.5 equivalents to potassium 2-keto-3-deoxy-D-gluconate). For this reason, this method is not satisfactory from the viewpoints of the safety or economical efficiency. Further, the water of 2000 weight times the amount of potassium 2-keto-3-deoxy-D-gluconate is used so that the volumetric efficiency is very bad. Thus, this method is not quite satisfactory as a method on industrial scale. On the other hand, the yield of a product, i.e., metasaccharic acid, was not described so that the yield is unclear.

### DISCLOSURE OF THE INVENTION

An object of the present invention is to provide a method for preparing 2-deoxyaldoses on industrial scale in which the yield is excellent and the operation is simple.

In order to achieve the above object, the present inventors have thought that 2-deoxyaldoses could be synthesized with the good yield when 3-deoxylaldonic acids such as metasaccharic acid or the like are obtained with the good yield by the unprotected reaction, resulting in extensive study on this matter.

As a result, they have found that 2-keto-3-deoxyaldonic acid which is available by the conventional technique is in a equilibrium with a lactone derivative under acidic conditions and it can be converted into 3-deoxyaldonic acids with the good yield when the catalytic hydrogenation is further carried out.

Meanwhile, when 2-keto-3-deoxy-D-gluconic acid (hereinafter referred to as KDG) as described in Carbohydr. Res., vol. 115, p. 288 (1983) is reduced using sodium borohydride, in order to make a preparation method available on industrial scale, the reaction is carried out by increasing the volumetric efficiency for enhancing the productivity. Then, it was found that great exothermicity was observed when adding sodium borohydride, making it difficult to secure safety. As a result of further extensive study, KDG is unstable to the heat under alkali conditions, which is proven to be one of the causes for worsening the yield. In order to overcome the above object, as a result of study, they have found that when a hydride reducing agent such as sodium borohydride or the like is fed in a divided manner or fed by dropping to suppress the reaction heat, then the reaction at high concentration can be proceeded with the good yield. Surprisingly, it was found that when the reaction at high concentration is carried out, it is possible to reduce a hydride reducing agent such as sodium borohydride or the like down to around 1 equivalent in terms of hydride. Due to the reduction of the amount of a hydride reducing agent used such as sodium borohydride or the like, there is a great advantage on the industrial production and there is not only an effect of the safety or economical efficiency, but also the by-product such as a boric acid and the like can be suppressed. Thus, a load to the follow-up process or the environment can be reduced. In this manner, they have found that, in a hydride reducing agent, 2-keto-3-deoxyaldonic acid can be converted into 3-deoxyaldonic acids with the good yield under unprotection in the same manner as the catalytic hydrogenation.

That is, the present invention is specified by the matters described in the following (1) to (11).
(1) A method for preparing a compound represented by the general formula (4) comprising the following two steps;
   a step of the reduction from a compound represented by the general formula (1) to a compound represented by the general formula (2) and/or the general formula (3), and
   a step of the decarboxylation from a compound represented by the general formula (2) and/or the general formula (3) to a compound represented by the general formula (4), 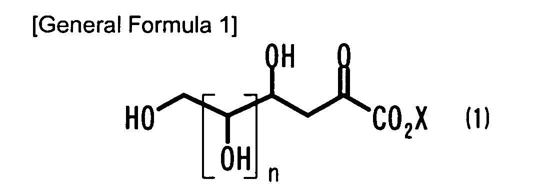 wherein X represents a hydrogen atom, an alkali metal or an alkali earth metal; and n represents 0 or 1, 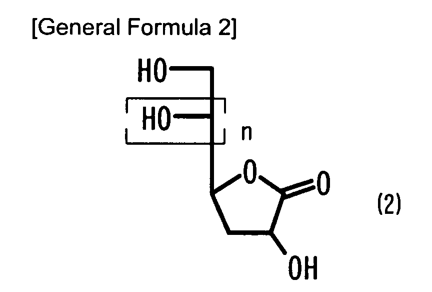 wherein n is the same as the above, 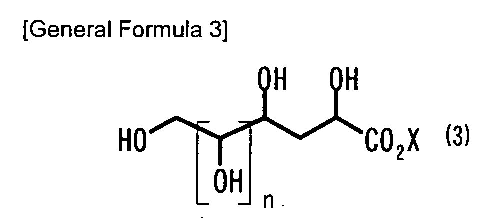 wherein X and n are the same as the above, 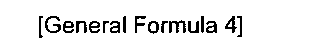 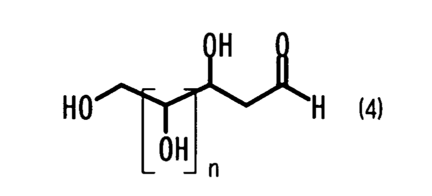 wherein n is the same as the above.
(2) The method as described in (1), wherein the reduction step is carried out by the catalytic hydrogenation.
(3) The method as described in (1), wherein the reduction step is carried out using a hydride reducing agent.
(4) The method as described in any one of (1) to (3), wherein both of the reduction step and the decarboxylation step are carried out in a water solvent.
(5) A method of reducing a compound represented by the general formula (1) to a compound represented by the general formula (2) and/or the general formula (3) by the catalytic hydrogenation, 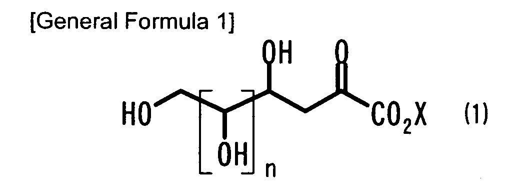 wherein X represents a hydrogen atom, an alkali metal or an alkali earth metal; and n represents 0 or 1, 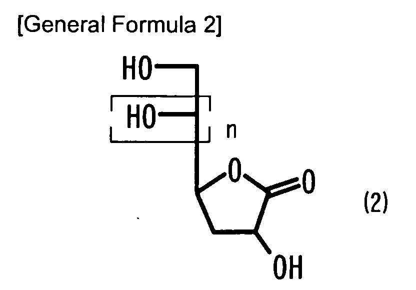 wherein n is the same as the above, 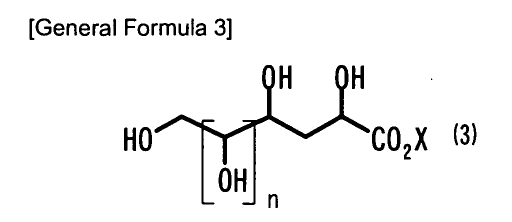 wherein X and n are the same as the above.
(6) The method as described in (5), wherein the catalytic hydrogenation is carried out under acidic conditions.
(7) The method as described in (6), wherein palladium loaded in an activated carbon is used for the catalytic hydrogenation.
(8) A method of reducing a compound represented by the general formula (1) to a compound represented by the general formula (2) [Chemical Formula 9] and/or (3) using a hydride reducing agent in a solvent of not more than 30 weight times the amount of a compound represented by the general formula (1), 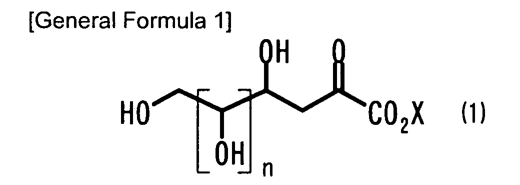 wherein X represents a hydrogen atom, an alkali metal or an alkali earth metal; and n represents 0 or 1, 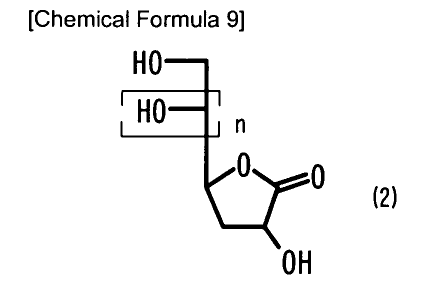 wherein n is the same as the above, 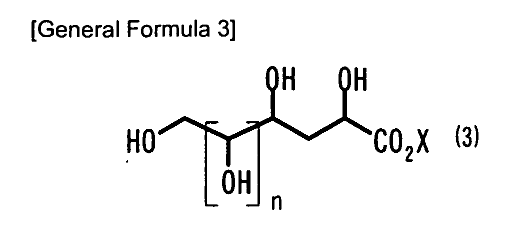 wherein X and n are the same as the above.
(9) The method as described in (8), wherein a reducing agent is fed in a divided manner or fed by dropping and the reaction is carried out at not more than 30°C.
(10) The method as described in (8) or (9), wherein sodium borohydride is used as a reducing agent.
(11) The method as described in any one of (5) to (10), wherein the reaction is carried out in a water solvent.

A metal loaded in an activated carbon (palladium or the like) is used for the reduction by the catalytic hydrogenation so that it is possible to easily recover and reuse the metal. On the other hand, in the reaction using a hydride reducing agent, even in a small amount of the reducing agent can result in the good yield. Accordingly, both of the reduction methods have advantages such that either of them is effective in the safety or economical efficiency and industrial waste is small.

Furthermore, even in a reaction solution containing 3-deoxyaldonic acids obtained by any of the methods, 3-deoxyaldonic acids can be obtained with the good yield in spite of the unprotected reaction so that a load to the next step of decarboxylation is reduced; therefore, 3-deoxyaldonic acids can be converted into 2-deoxyaldoses with the good yield even without a specific purifying operation.

Meanwhile, the method according to the present invention has advantages such that the volumetric efficiency is superior, both of the reduction step and decarboxylation step are carried out in a water solvent and the like. For this reason, as the method is superior in the economical efficiency, safety and productivity, it is useful as a preparation method on industrial scale.

Furthermore, the method according to the present invention proceeds with the reaction in the same manner even in 2-keto-3-deoxyxylonic acid or the like which has carbon atoms one shorter than 2-keto-3-deoxygludconic acid. There is applicability as a preparation method of 2-keto-3-deoxyaldonic acids or 2-deoxytetroses having 5 carbon atoms which is useful as medical supplies or intermediate raw materials.

The present invention is to effectively synthesize 3-deoxylaldonic acids by a process of the catalytic hydrogenation or a reduction process using a hydride reducing agent of 2-keto-3-deoxyaldonic acid which is synthesizable according to a known method under unprotection, and then convert the 3-deoxylaldonic acids into 2-deoxyaldoses by the decarboxylation step. The most important feature of the present invention is to prepare 2-deoxyaldoses with the good yield and a simple operation.

According to the present invention, 2-deoxyaldoses can be obtained with the good yield by the simple operation. That is, 3-deoxyaldonic acids are obtained with high yield by the reduction by the catalytic hydrogenation or the reduction using a hydride reducing agent, then the 3-deoxyaldonic acids can be converted into 2-deoxyaldoses without purifying through the decarboxylation step. This method has an advantage as a method on industrial scale.

### BEST MODE FOR CARRYING OUT THE INVENTION

The present invention will be described in more detail below.

2-keto-3-deoxyaldonic acid represented by the general formula (1) of a starting raw material is described below.

In the general formula (1), an alkali metal and an alkali earth metal in X are not particularly restricted. Concrete examples of the alkali metal include lithium, natrium, kalium and the like. Concrete examples of the alkali earth metal include magnesium, calcium, barium and the like.

The stereo-structure of a hydroxyl group is not particularly restricted. For example, when n is 1, 2-keto-3-deoxygluconic acid, 2-keto-3-deoxymannonic acid, 2-keto-3-deoxygalactonic acid, 2-keto-3-deoxygulonic acid, 2-keto-3-deoxyidonic acid, 2-keto-3-deoxytalonic acid, 2-keto-3-deoxyallonic acid and 2-keto-3-deoxyaltronic acid can be cited. When n is 0, 2-keto-3-deoxyxylonic acid and the like can be cited. The stereo-structure of a hydroxyl group may be either D series structure or L series structure.

A compound represented by the general formula (1) can be obtained by known methods such as 1) a dehydration reaction by an enzyme, a microorganism or the like from a raw material such as gluconic acid, xylonic acid, arabinonic acid, fuconic acid, galactonic acid and the like according to a method described in Methods Enzymol., vol. 41, p. 99, Methods Enzymol., vol. 42, p. 301 or the like, 2) an oxidation reaction by an enzyme described in Carbohydr. Res., vol. 115, p. 288 (1983) or the like, 3) an aldol reaction by an enzyme described in J. Am. Chem. Soc., vol. 118, p. 2117 (1996) or the like and 4) a synthetic chemical method using a protecting group described in J. Carbohydro. Chem. vol. 10, p. 787 (1991), Carbohydro. Res., vol. 275, p. 107 (1995) or the like.

Furthermore, the method according to the present invention can be carried out in a water solvent so that, for example, an aqueous solution containing a compound represented by the general formula (1) obtained by the enzyme reaction or the like can be directly provided, i.e., without conducting an operation such as isolation or the like, as a reaction raw material of the present invention, or it can also be provided as a reaction raw material of the present invention without conducting an operation such as isolation or the like after carrying out a treatment such as the removal of protein as required.

Furthermore, a compound represented by the general formula (1), for example, 2-keto-3-deoxy-D-gluconic acid has been known as having an equilibrium state of a ring structure in which a hydroxyl group and a carbonyl group in the compound are condensed in a solution (described in J. Carbohydro. Chem., vol. 10, p. 787 (1991)). In the present invention, isomers of this ring structure are also included.

Next, the catalytic hydrogenation method in the reduction step is described below.

As a metal of a metal catalyst for use in the catalytic hydrogenation method, there can be exemplified, for example, palladium, rhodium, ruthenium, platinum, nickel (raney nickel) and the like. These metals may be used as a metal catalyst as it is or may be used in the form of a salt such as metal oxide, metal chloride and the like as a metal catalyst.

The amount of a metal catalyst in use is not particularly restricted as far as the reaction can proceed. From the economical aspect, it is preferably from 0.1 to 30 weight % to the compound represented by the general formula (1).

In consideration of reuse of a metal catalyst, a metal loaded in a carrier is preferably used as the metal catalyst for use in the present invention. When a metal catalyst in which a metal is loaded in a carrier is used, as a carrier in use, there can be mentioned, for example, activated carbon, SiO₂, Al₂O₃, BaSO₄, TiO₂, ZrO₂, MgO, ThO₂, diatomite and the like. The amount of a metal loaded in a carrier may be arbitrary, but it is in the range of 0.1 to 30 weight % to the carrier.

From the economical efficiency, easiness of availability and the like, a preferred example of a catalyst includes palladium loaded in an activated carbon.

In the catalytic hydrogenation of the present invention, the hydrogen pressure in the reactive system is not particularly restricted, and may be in an atmospheric pressure or in a pressure application.

A reaction temperature of the catalytic hydrogenation reaction is not less than 20°C and not more than the boiling point of a solvent, and preferably not less than 40°C and not more than the boiling point of a solvent.

The solvent used in the catalytic hydrogenation reaction is not particularly restricted as far as the above reaction can proceed. However, it is preferably a solvent dissolving a compound represented by the general formula (1). Examples thereof include ethers such as water, alcohols, dioxane, tetrahydrofuran and the like, and may contain water arbitrarily.

The catalytic hydrogenation reaction of a compound represented by the general formula (1) is preferably carried out under acidic conditions. Acidity is adjusted by addition of carboxylic acid residue (in case of X = H) of the general formula (1) or acids to be described later. As acids to be used, there can be mentioned, for example, organic acids, inorganic acids and cation exchange resins. Concrete examples of the organic acid include methane sulfonic acid, trifluoromethane sulfonic acid, p-toluene sulfonic acid, acetic acid, trifluoroacetate and the like. Examples of the inorganic acid include hydrochloric acid, sulfuric acid, phosphoric acid and the like. Furthermore, the reaction rate may is accelerated by addition of the above organic acid or the inorganic acid in some cases. The amount thereof used may be added such that liquidity of the reaction solution has pH of not more than 5 and preferably pH of not more than 3.

After the catalytic hydrogenation is completed, as 3-deoxyaldonic acids, a mixture of a compound represented by the general formula (2) and a compound represented by the general formula (3) partially hydrolyzed is obtained in some cases. Furthermore, in the general formula (2), other than 1,4-lactone, 1,5-lactone is also included.

In a compound represented by the general formula (2), the stereo-structure of a hydroxyl group at □ position is not particularly restricted. For example, the stereo selectivity as shown in the formula (5) is obtained in some cases. This is considered to be caused by the steric hindrance of a lactone-ring substituent. However, this does not influence on the yield of the decarboxylation reaction of the follow-up process or the like.

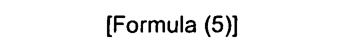

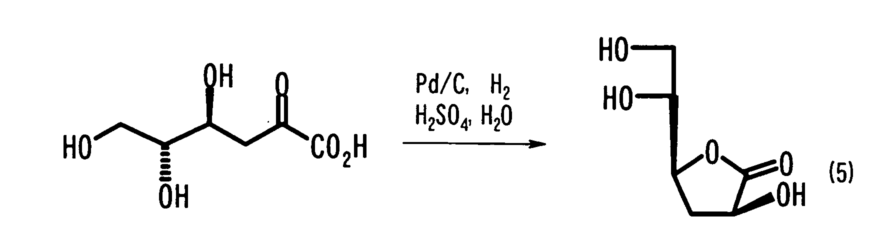

Further, a compound represented by the general formula (1) under acidic conditions is in equilibrium of lactonization represented by the formula (6), so the catalytic hydrogenation reaction can be carried out after it is synthesized to a lactone derivative.

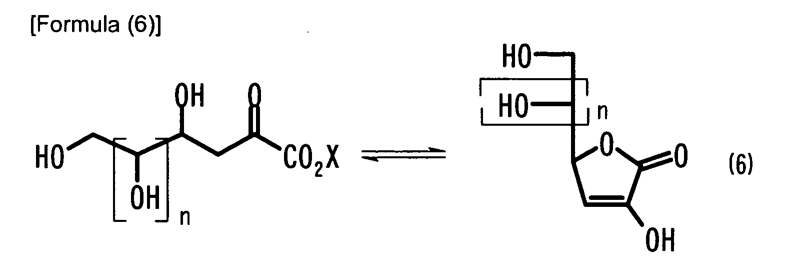

For example, a lactone derivative can be synthesized simply by heating a compound represented by the general formula (1) at a temperature of from 40°C to the boiling point of a solvent under acidic conditions. At that time, the lactone derivative can be synthesized with the good yield by removing the solvent under a reduced pressure.

The solvent used in this reaction is preferably a solvent dissolving a compound represented by the general formula (1) and examples thereof include those as described before. In a reaction for carrying out the catalytic hydrogenation of the lactone derivative for a compound represented by the general formula (2), a metal and the pressure of hydrogen in use are the same as the above method. In this case, liquidity of the catalytic hydrogenation reaction is not particularly restricted as far as the reaction can proceed. However, a compound represented by the lactone derivative in alkalinity is hydrolyzed to be a compound represented by the general formula (1), resulting in reducing the yield. Under neutral or acidic conditions are preferable. Under acidic conditions, acidification can be carried out in an organic acid or inorganic acid. As an acid to be used for carrying out the above reaction under acidic conditions, the above acids can be cited. A reaction temperature in this case is not particularly restricted, and it is not more than the boiling point of a solvent and preferably not less than 10°C and not more than the boiling point of a solvent.

The reduction using a hydride reducing agent in the reduction process is described below. As the hydride reducing agent, there can be exemplified, for example, an aluminum hydride compound and/or boron hydride compound and the like. As the boron hydride compound, there can be exemplified, for example, alkali borohydrides such as sodium borohydride, potassium borohydride and the like, and sodium borohydride is preferable from the viewpoints of the economical efficiency or easiness of handling.

The solvent is not particularly restricted as far as the reaction can proceed. It is preferably a solvent dissolving a compound represented by the general formula (1). Water is usually used for the solvent from the viewpoints of safety and economical efficiency. Further, protic solvents such as other alcohols can be used and contain water arbitrarily

As for the amount of the reducing agent used, the upper limit is not particularly restricted as far as it is not less than 1 equivalent (for example, 0.25 mole equivalent in case of sodium borohydride) to a compound represented by the general formula (1) in terms of a hydride. It is preferably not less than 1 equivalent and not more than 4 equivalents (not less than 0.25 mole equivalent and not more than 1 mole equivalent in case of sodium borohydride) and more preferably not less than 1 equivalent and not more than 2 equivalents (not less than 0.25 mole equivalent and not more than 0.5 mole equivalent in case of sodium borohydride).

The shape of a reducing agent, for example sodium borohydride is not particularly restricted. Commercial products such as powder products, granular products, those dissolved in a 40% sodium hydroxide aqueous solution and the like can be used as intact.

The weight multiple times of water is a value to the weight of X in the general formula (1) in terms of hydrogen. From the operational aspect, the lower limit is not less than 2 times, while the upper limit is preferably not more than 30 times and more preferably not more than 15 times.

A reaction temperature sets the lower limit at a temperature in which the aqueous solution is not frozen. The upper limit is different depending on pH of the reaction solution. Specifically, when pH is not less than 7 and not more than 11, the reaction temperature is preferably not more than 50°C. More appropriately, it is not more than 30°C. Furthermore, when pH is greater than 11, it is preferably not more than 30°C and more preferably not more than 15°C. Furthermore, when the reaction temperature is not more than 15°C, the upper limit does not depend on pH. The reaction temperature can be controlled, for example, by feeding in a divided manner when sodium borohydride powder is used and by the dropping rate in case of a liquid product, in addition to cooling the surrounding of the reaction vessel.

As the reaction product by the hydride reducing agent, 3-deoxyaldonic acid represented by the general formula (3) is obtained, while the stereo-structure of a hydroxyl group at □ position is not particularly restricted.

Below is described a method for synthesizing a compound represented by the general formula (4) from a compound represented by the general formula (2) and/or the general formula (3) by Ce (III) in the decarboxylation step.

In case the reduction process is the catalytic hydrogenation, a metal catalyst is removed after the reaction is completed. A compound represented by the general formula (2) and/or the general formula (3) can be converted into a compound represented by the general formula (4) using Ce (III) such as Ce₂(SO₄)₃ without purifying any longer. When water is used in a solvent for the catalytic hydrogenation, the reaction can be continuously carried out in a water solvent without requiring a solvent replacement. Ce (III) in use is not less than 2 equivalents, and preferably not less than 2 equivalents and not more than 5 equivalents from the economical aspect. At this time, a sulfuric acid is preferably added in the amount of not less than 2 equivalents to Ce (III). The reaction temperature at this time is from 20 to 70°C.

Next is described a method for obtaining a compound represented by the general formula (4) from a compound represented by the general formula (2) and/or the general formula (3) by the hydroxyl radical in the decarboxylation step.

A compound represented by the general formula (2) after the completion of the catalytic hydrogenation is partially hydrolyzed and obtained as a mixture with a compound represented by the general formula (3) in some cases. At that time, it can be converted into a compound represented by the general formula (3) by using an inorganic base.

As the inorganic base to be used for the hydrolysis, there can be exemplified, for example, lithium hydroxide, sodium hydroxide, sodium hydrogen carbonate, sodium carbonate, potassium hydroxide, potassium hydrogen carbonate, potassium carbonate, calcium hydroxide and the like. The equivalent to be used is not particularly restricted. However, it is preferably from 0.5 to 3 equivalents.

The reaction temperature during hydrolysis is not particularly restricted. However, it is preferably from -10 to 70°C.

When a compound represented by the general formula (1) is reduced using hydride reducing agent, a compound represented by the general formula (3) is obtained. Thus, a compound represented by the general formula (3) can be used in the decarboxylation step to be described below without purification as intact.

A compound represented by the general formula (3) can be converted into a compound represented by the general formula (4) by the reaction with the hydroxyl radical.

The hydroxyl radical can be generated by the reaction of a metal with hydrogen peroxide water. Concrete examples of the metal include Fe (II), Fe (III), Ti(III), Ti (IV), Cu (I), Cu (II) and the like. The equivalent to be used is not particularly restricted as far as the reaction can proceed. However, it is from 0.1 to 50 mole %.

The amount of hydrogen peroxide in use is not particularly restricted as far as the reaction can proceed. However, it is preferably from 1 to 10 equivalents and more preferably from 2 to 5 equivalents.

As 2-deoxyaldoses represented by the general formula (4) obtained in the decarboxylation step, there can be exemplified, for example, 2-deoxypentoses such as D-2-deoxyribose or L-2-deoxyribose, D-2-deoxyxylose, L-2-deoxyxylose or the like, and 2-deoxytetroses such as (3R)-3,4-dihydroxybutanal, (3S)-3,4-dihydroxybutanal or the like.

### EXAMPLES

The present invention is described specifically below by way of Examples. However, the present invention is not restricted to these Examples.

### Example 1

### Catalytic Hydrogenation of 2-keto-3-deoxy-D-gluconic Acid

50 mg of 10% palladium carbon (48% water-containing product) was added to 5.0 g of a 10% aqueous solution of 2-keto-3-deoxy-D-gluconic acid (hereinafter referred to as KDG) and the resulting mixture was heated to an internal temperature of 48°C. The solution was reacted under a hydrogen flow for 9 hours. Then, the reaction solution was analyzed according to HPLC. As a result, metasaccharic acid lactone was obtained with the reaction yield of 54%, while metasaccharic acid was obtained with the reaction yield of 26%.

HPLC Analytic Conditions: ShodexAsahipack NH2-P50 (manufactured by Showa Denko K.K.), 50 mM sodium hydrogen phosphate aqueous solution, flow rate of 1 ml/min. and detection UV of 210 nm.

### Example 2

### Catalytic Hydrogenation of KDG

The reaction was carried out in the same manner as in Example 1, except that 45 µl of sulfuric acid was added. The reaction solution was analyzed according to HPLC. As a result, a mixture of metasaccharic acid lactone and metasaccharic acid was obtained with the reaction yield of 92%.

### Example 3

### Catalytic Hydrogenation of KDG

The reaction was carried out in the same manner as in Example 1, except that 107 µl of sulfuric acid was added. The reaction solution was analyzed according to HPLC. As a result, a mixture of metasaccharic acid lactone and metasaccharic acid was obtained with the reaction yield of 96%.

### Example 4

### Catalytic Hydrogenation of KDG

0.19 g of 10% palladium carbon (48% water-containing product) was added to 6.3 g of a 30% aqueous solution of KDG and the resulting mixture was heated to an internal temperature of 48°C. The solution was reacted under a hydrogen flow for 20 hours. Then, the reaction solution was analyzed according to HPLC. As a result, a mixture of metasaccharic acid lactone and metasaccharic acid was obtained with the reaction yield of 84%.

### Example 5

### Catalytic Hydrogenation of KDG

The reaction was carried out in the same manner as in Example 4, except that 178 µl of sulfuric acid was added. The reaction solution was analyzed according to HPLC. As a result, a mixture of metasaccharic acid lactone and metasaccharic acid was obtained with the reaction yield of 89%.

### Example 6

### Catalytic Hydrogenation of KDG Sodium Salt

0.6 g of sulfuric acid and 0.10 g of 10% palladium carbon (48% water-containing product) were added to 5.0 g of an aqueous solution containing 4.66 mmole of KDG sodium salt, and the resulting mixture was heated to an internal temperature of 48°C. The solution was reacted under a hydrogen flow for 20 hours. Then, the reaction solution was analyzed according to HPLC. As a result, a mixture of metasaccharic acid lactone and metasaccharic acid was obtained with the reaction yield of 80%.

### Example 7

### Catalytic Hydrogenation of KDG Potassium Salt

The reaction was carried out in the same manner as in Example 6, except that KDG potassium salt was used instead of KDG sodium salt. The reaction solution was analyzed according to HPLC. As a result, it was found that a mixture of metasaccharic acid lactone and metasaccharic acid was obtained with the reaction yield of 82%.

### Example 8

### Reuse of Palladium Carbon

120 µl of sulfuric acid and 80 mg of 10% palladium carbon (48% water-containing product) were added to 16 g of a 4.4% aqueous solution of KDG, and the resulting mixture was reacted under a hydrogen flow. Then, the reaction solution was analyzed according to HPLC. As a result, a mixture of metasaccharic acid lactone and metasaccharic acid was obtained with the yield of 77%. Palladium carbon used in this reaction was reused (for the first time) to carry out the same reaction. The reaction solution was analyzed according to HPLC. As a result, it was found that a mixture of metasaccharic acid lactone and metasaccharic acid was obtained with the yield of 72%. Then, the same reaction was repeated. The reaction yield was 78% when palladium carbon was used for the second time, while it was 75% when palladium carbon was used for the third time. Thus, it was confirmed that palladium carbon could be reused.

### Example 9

### Catalytic Hydrogenation of 5-(1,2-dihydroxyethyl)-3-hydroxy-5H-furan-2-one

5.0 g of 2 normal hydrochloric acid aqueous solution was added to 5.0 g of an aqueous solution containing KDG potassium salt (4.77 mmole) and the resulting mixture was reacted at 80°C for 2 hours. Subsequently, condensation in a reduced pressure was carried out. Then, as a result of analysis according to HPLC, it was confirmed that 5-(1,2-dihydroxyethyl)-3-hydroxy-5H-furan-2-one was generated. 5.0 g of water and 0.2 g of 10% palladium carbon (48% water-containing product) were added thereto and the resulting mixture was reacted under a hydrogen flow. Then, a mixture of metasaccharic acid lactone and metasaccharic acid was obtained with the reaction yield of 61 %.

### Example 10

### Synthesis of D-2-deoxyribose

Palladium carbon contained in the reaction solution obtained in Example 1 was filtered out from the reaction solution. Then 1.8 g of cerium (IV) sulfate tetrahydrate and 0.87 g of sulfuric acid were added to 20 ml of water at 37°C and the resulting mixture was dropped. After the reaction was completed, the reaction solution was analyzed according to HPLC. As a result, the total yield from KDG was 51%.

### Example 11

### Synthesis of D-2-deoxyribose

0.6 g of sulfuric acid and 0.1 g of 10% palladium carbon (48% water-containing product) were added to an aqueous solution containing KDG potassium salt (5.73 mmole), and the resulting mixture was heated to 50°C and reacted under a hydrogen flow. After the reaction was completed, palladium carbon was filtered out, and 0.67 g of calcium carbonate was added thereto. A precipitate was filtered out and 0.42 g of calcium hydroxide was added to the filtrate. Furthermore, carbonic gas was blown thereinto and heated to 100°C. Then, the precipitate was filtered out. A mixture of 1 ml of water, 9.6 mg of iron (II) sulfate heptahydrate and 8.8 g of barium acetate which was prepared in advance was added to the filtrate. The resulting solution was heated to 50°C. Further, 0.4 g of 30% hydrogen peroxide water was added three times at an interval of 30 minutes. After the reaction was completed, as a result of the HPLC analysis, D-2-deoxyribose was obtained with the reaction yield of 47%. From the generated product, inorganic salt was filtered out, followed by addition of 0.33 g of aniline to synthesize 2-deoxy-N-phenyl-D-rebosilamine for analysis (the total yield from KDG of 30%). The analysis values are shown below.
¹H NMR (DMSO): 1.7 to 1.9 (2H, m), 3.4 to 3.7 (4H, m), 4.39 (1 H, d), 4.6 to 4.7 (2H, m), 6.38 (1H, d), 6.5 to 6.7 (3H, m), 7.0 to 7.1 (2H, m)

### Example 12

### Catalytic Hydrogenation of 2- keto-3-deoxy-D-xylonic Acid

The reaction was carried out in the same manner as in Example 7, except that sodium salt of 2- keto-3-deoxy-D-xylonic acid was used instead of KDG potassium salt. Subsequently, the reaction solution was treated with an acidic ion exchange resin (Amber Light IR-120PLUS). Then, thereto was added 0.55 equivalent of calcium hydroxide, followed by stirring for 2 hours. Then, carbonic gas was ventilated until the resulting reaction solution was neutralized. After the heat filtering, the filtrate was concentrated to obtain calcium salt of (4S)-2,4,5-trihydroxy-pentanoic acid with the yield of 81 %. The analysis values are shown below.
¹H NMR (D₂O): 1.5 to 1.8 (2H, m), 3.2 to 3.5 (2H, m), 3.74 (1 H, m), 4.06 (1 H, m)

### Example 13

### Synthesis of 3-deoxy-D-arabino-hexonic Acid Calcium

0.42 g of 10% palladium carbon (48% water-containing product) was added to 14 g of an aqueous solution containing KDG (23.4 mmole), and the resulting mixture was heated to an internal temperature of 48°C and reacted under a hydrogen flow. Then, a catalyst was filtered out. Thereto was added calcium hydroxide (2 g) and the mixture was reacted for an hour. Then, carbonic gas was flown for neutralization. After the heat filtering, a precipitated solid was obtained and dried to obtain 4.2 g of the entitled compound. The measurement results of specific rotation are shown below.
Measurement value: [□]_{D}²³ = -22.5° (c 0.303, H₂0)
Literature value; [□]_{D}²⁰ = -22° [Acta. Chem. Scand., vol. 35, p. 155 (1981)]
Other physical properties values are shown below.
¹H NMR (D₂O): 1.7 to 1.8 (2H, m), 3.4 to 3.7 (4H, m), 4.0 to 4.1 (1H, m).
¹³C NMR (D₂O): 38.12, 63.52, 69.75, 70.49, 75.86, 182.93.

### Example 14

### Reduction of KDG using Sodium Borohydride and Stability of KDG

1.21 g (5.61 mmole) of KDG potassium salt was dissolved in 10 g of water (10 times the amount of KDG). pH of the solution was 7.3. Subsequently, when 64 mg (1.69 mmole: 0.3 equivalent) of granular sodium borohydride was added at 25°C, the reaction temperature increased to 33°C. Furthermore, pH after the completion of the reaction was 10.8. After the reaction was completed, when observed by HPLC, the reaction yield of metasaccharic acid was 94%. The reaction heat was observed as remarkable. The generated product, i.e., metasaccharic acid is stable, whereas KDG shows different stability depending on pH and the temperature. In order to carry out the reaction with the good yield, stability to the heat of KDG was measured in the presence of water with 10 times the amount of KDG. (Table 1) (Table 2) (Table 3)

**[Table 1]**

| Remaining Rate of KDG at pH 7.0 (observed according to HPLC) | | |
|---|---|---|
| Hours | 25°C | 50° C |
| 2 | 101% | 100% |
| 4 | 100% | 101% |
| 21 | 100% | 100% |
| 24 | 101% | 99% |

**[Table 2]**

| Remaining Rate of KDG at pH 8.5 (observed according to HPLC) | | |
|---|---|---|
| Hours | 25°C | 50° C |
| 2 | 100% | 101% |
| 4 | 99% | 100% |
| 21 | 101% | 99% |
| 24 | 100% | 98% |

**[Table 3]**

| Remaining Rate of KDG at pH 10.5 (observed according to HPLC) | | |
|---|---|---|
| Hours | 25°C | 50° C |
| 2 | 102% | 101% |
| 4 | 101% | 99% |
| 21 | 100% | 94% |
| 24 | 101% | 93% |

From these results, it was found that, in the range of 7.0 to 10.5 of pH, the reaction could be stably carried out.

### Comparative Example 1

13 mg (0.35 mmole: 0.3 equivalent) of sodium borohydride was added to 500 g of KDG potassium salt 0.25 g (1.16 mmole)-dissolved aqueous solution (dissolved in water with 2000 times the amount of KDG) which was prepared in the same manner as in Carbohydr. Res., vol. 115, p. 288 (1983) at 25°C. When observed by HPLC, the reaction yield of metasaccharic acid was 36%.

### Example 15

### Reduction of KDG at pH of not less than 7 and not more than 11 using Sodium Borohydride

9.95 g (55.85 mmole) of KDG was dissolved in 30 g of water. pH of the solution was adjusted to 8.6 with a 40 weight % sodium hydroxide aqueous solution under ice-cold conditions. Subsequently, 634 mg (16.76 mmole: 0.3 equivalent) of granular sodium borohydride was added three time in a divided manner. At this time, the reaction temperature was controlled to not more than 15°C. pH after the completion of the reaction was 10.3. When observed by HPLC, the reaction yield of metasaccharic acid was 95%.

### Example 16

### Reduction of KDG at pH of not less than 11 using Sodium Borohydride and KDG Stability

The reaction was carried out by controlling the reaction temperature to not more than 15°C in the same manner as in Example 15, except that pH was adjusted to 12.5 with a 40 weight % sodium hydroxide aqueous solution. Furthermore, pH after the completion of the reaction was 13.1. When observed by HPLC, the reaction yield of metasaccharic acid was 96%.

Here, the results of stability test to the KDG heat in the presence of water 10 times the amount of KDG are shown below. (Table 4)

**[Table 4]**

| Remaining Rate of KDG at pH 12.0 (observed according to HPLC) | | | |
|---|---|---|---|
| Hours | 4°C | 25°C | 50°C |
| 2 | 101% | 99% | 61% |
| 4 | 99% | 95% | 56% |
| 21 | 100% | 84% | - |
| 24 | 98% | 79% | - |

From these results, it was found that KDG was remarkably decomposed at 50°C. So, when pH in the reaction was more than 11, as far as the reaction temperature was maintained at not more than 25°C, it was found that metasaccharic acid was obtained with the good yield like in this Example.

### Example 17

### Reduction of KDG using Sodium Borohydride (a liquid product)

The reaction was carried out by controlling the reaction temperature to not more than 15°C in the same manner as in Example 15, except that a 40 weight % sodium hydroxide aqueous solution containing 12 weight % sodium borohydride was dropped instead of feeding granular sodium borohydride three times in a divided manner. pH after the reaction was not less than 14.0. When observed by HPLC, the reaction yield of metasaccharic acid was 95%.

### Example 18

### Reduction of KDG using Sodium Borohydride

1.0 g of KDG (5.61 mmole) was dissolved in 10 g of water (10 times). pH was adjusted to 4.3 with a 40 weight % sodium hydroxide aqueous solution. Subsequently, 106 mg (3.37 mmole: 0.5 equivalent) of granular sodium borohydride was added thereto. The reaction temperature was from 24 to 30°C. Meanwhile, pH after the completion of the reaction was 10.4. When observed by HPLC, the reaction yield of metasaccharic acid was 94%.

### Example 19

### Reduction of 2-keto-3-deoxy-D-xylonic Acid using Sodium Borohydride

The reaction was carried out in the same manner as in Example 13, except that 2-keto-3-deoxy-D-xylonic acid was used instead of KDG potassium salt. From the HPLC analysis, the reaction yield of (4S)-2,4,5-trihydroxy-pentanoic acid was 93%. The analysis results of a solid obtained by condensation of the reaction solution are shown below.

¹H NMR (D₂O): 1.5 to 2.0 (2H, m), 3.3 to 3.8 (2H, m), 3.8 to 3.9 (1H, m), 4.0 to 4.2 (1H, m).

## Claims

1. A method for preparing a compound represented by the general formula (4) comprising the following two steps;
a step of the reduction from a compound represented by the general formula (1) to a compound represented by the general formula (2) and/or the general formula (3), and
a step of the decarboxylation from a compound represented by the general formula (2) and/or the general formula (3) to a compound represented by the general formula (4), 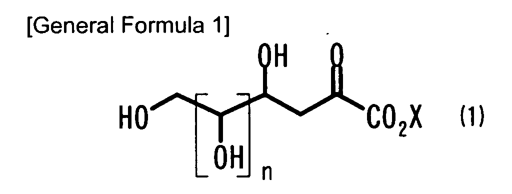 wherein X represents a hydrogen atom, an alkali metal or an alkali earth metal; and n represents 0 or 1, 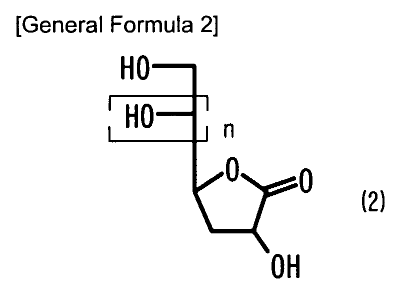 wherein n is the same as the above, 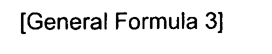 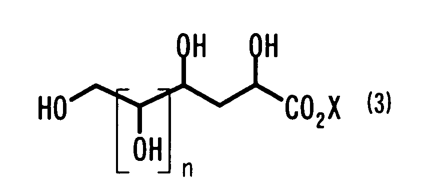 wherein X and n are the same as the above, 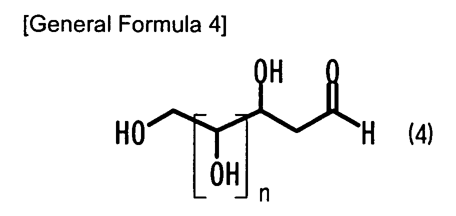 wherein n is the same as the above.

2. The method according to claim 1, wherein the reduction step is carried out by the catalytic hydrogenation.

3. The method according to claim 1, wherein the reduction step is carried out using a hydride reducing agent.

4. The method according to any one of claims 1 to 3, wherein both of the reduction step and the decarboxylation step are carried out in a water solvent.

5. A method of reducing a compound represented by the general formula (1) to a compound represented by the general formula (2) and/or the general formula (3) by the catalytic hydrogenation, 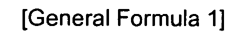 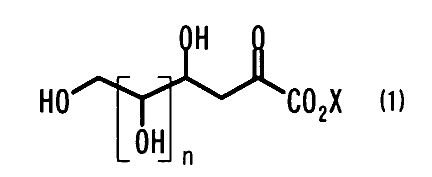 wherein X represents a hydrogen atom, an alkali metal or an alkali earth metal; and n represents 0 or 1, 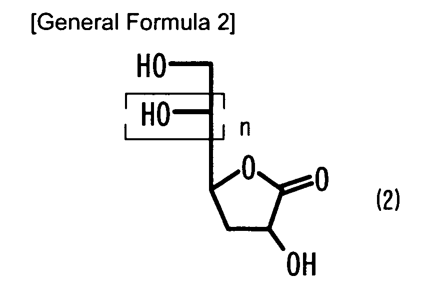 wherein n is the same as the above, 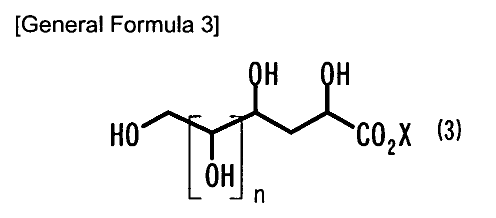 wherein X and n are the same as the above.

6. The method according to claim 5, wherein the catalytic hydrogenation is carried out under acidic conditions.

7. The method according to claim 6, wherein palladium loaded on an activated carbon is used for the catalytic hydrogenation.

8. A method of reducing a compound represented by the general formula (1) to a compound represented by the general formula (2) and/or (3) using a hydride reducing agent in a solvent of not more than 30 weight times the amount of a compound represented by the general formula (1), 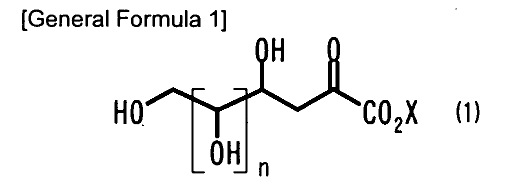 wherein X represents a hydrogen atom, an alkali metal or an alkali earth metal; and n represents 0 or 1, 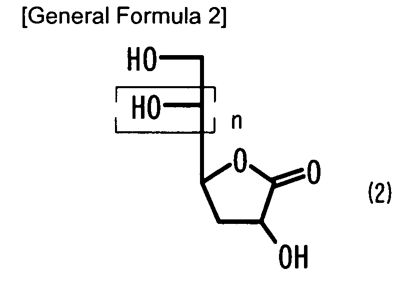 wherein n is the same as the above, 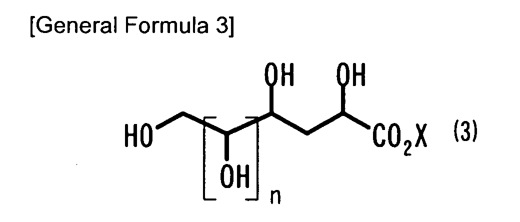 wherein X and n are the same as the above.

9. The method according to claim 8, wherein a reducing agent is fed in a divided manner or fed by dropping and the reaction is carried out at not more than 30°C.

10. The method according to claim 8 or 9, wherein sodium borohydride is used as a reducing agent.

11. The method according to any one of claims 5 to 10, wherein the reaction is carried out in a water solvent.
